# EUROPÄISCHE PATENTSCHRIFT

(11) **EP 0 891 376 B1**
(45) Veröffentlichungstag und Bekanntmachung des Hinweises auf die Patenterteilung: **02.06.2004**
(21) Anmeldenummer: 97915428.3
(22) Anmeldetag: 25.03.1997
(51) Int. Cl.: C07K 7/02, A61K 38/10, C12P 21/02, C12N 1/14

(54) **ANTIFUNGISCHE PEPTIDE AUS SCLERODERMA TEXENSE**
ANTIFUNGAL PEPTIDES FROM SCLERODERMA TEXENSE
PEPTIDES ANTIFONGIQUES EXTRAITS DE SCLERODERMA TEXENSE

(30) Priorität: 01.04.1996 DE 19612805
(43) Veröffentlichungstag der Anmeldung: 20.01.1999
(73) Patentinhaber: Aventis Pharma Deutschland GmbH, 65929 Frankfurt am Main (DE)
(72) Erfinder: STUMP, Heike, D-61184 Karben (DE); STAHL, Wilhelm, D-65510 Idstein (DE); WINK, Joachim, D-63322 Rödermark (DE); MARKUS, Astrid, D-65835 Liederbach (DE); KOGLER, Herbert, D-61479 Glashütten (DE); BACKHAUS, Jürgen, D-68535 Edingen (DE)
(86) Internationale Anmeldenummer: PCT/EP1997/001507
(87) Internationale Veröffentlichungsnummer: WO 1997/036921

(56) Entgegenhaltungen:
- EP-A- 0 622 375
- QUARTERLY REVIEWS OF BIOPHYSICS, Bd. 26, Nr. 4, November 1993, Seiten 365-421, XP002034867 M.S.P. SANSOM: "Structure and function of channel-forming peptaibols"
- JOURNAL OF ANTIBIOTICS, Bd. 41, Nr. 6, Juni 1988, TOKYO JP, Seiten 814-818, XP002034868 T. FUJITA ET AL.: "Structural Elucidation of Trichosporin-B-Ia, IIIa, IIId and V from Trichoderma Polysporum" in der Anmeldung erwähnt

### SEQUENZ PROTOKOLL

(1) ALLGEMEINE INFORMATION:
   (i) ANMELDER:
      (A) NAME: Hoechat Aktiengesellschaft
      (B) STRASSE: -
      (C) ORT: Frankfurt
      (D) BUNDESLAND: -
      (E) LAND: Deutschland
      (F) POSTLEITZAHL: 65926
      (G) TELEPHON: 069-305-5307
      (H) TELEFAX: 069-35-7175
      (I) TELEX: -
   (ii) ANMELDETITEL: Antifungische Peptide aus Scleroderma texense
   (iii) ANZAHL DER SEQUENZEN: 1
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 21 Aminosäuren
      (B) ART: Aminosäure
      (C) STRANGFORM: Einzel
      (D) TOPOLOGIE: linear
   (ii) ART DES MOLEKÜLS: Peptid
   (ix) MERKMALE:
      (A) NAME/SCHLÜSSEL: Peptid
      (B) LAGE: 1..21
      (D) SONSTIGE ANGABEN: /label=
         /note= "2-5 Xaa is Aib; 8 Xaa in Aib; 9 Ala is bAla; 11 Xaa is Aib; 12 Als is bAla; 14 Xaa is Aib; 15 Ala is bAla; 17-19 Xaa is Aib; 21 Arg is Argininol"
   (xi) SEQUENZBESCHREIBUNG: SEQ ID NO: 1:
   Pro Xaa Xaa Xaa Xaa Ala Ala Xaa Ala Leu Xaa Ala Ala Xaa Ala Ala
   1               5                   10                  15
   Xaa Xaa Xaa Ala Arg
               20

## Beschreibung

Die Erfindung betrifft neue Peptide, nämlich Texenomycene, aus Scleroderma texense, isbesondere die Peptide Texenomycin A und B, ein Verfahren zu deren Herstellung und ihre Verwendung als Arzneimittel, insbesondere als Antimykotikum.

Die Texenomycine gemäß der vorliegenden Erfindung zeichnen sich im besonderen durch die Aminosäuresequenz I aus, worin bedeuten
FS einen Oxo-Fettsäurerest,
Aib Aminoisobuttersäure und
Arg-o1 = Argininol.

Der Fettsäurerest besteht aus 3 bis 20, vorzugsweise aus 8 bis 15 Kohlenstoffatomen.

Vorzugsweise befindet sich die Oxo-Gruppe des Fettsäurerestes in 3-Position.

Verbindungen, die den Texenomycinen entsprechen, sind bis heute in der Literatur noch nicht beschrieben. Strukturell vergleichbare Verbindungen, wie die kürzlich beschriebenen Trichosporine (vgl. T. Fujita et al., J.Antibiotics (1988), 41, 814) unterscheiden sich aber schon allein durch die geringere Zahl von Aib-Resten sowie durch die Aminosäuresequenz.

Besonders bevorzugt weist der Fettsäurerest der erfindungsgemäßen Peptide die Konstitutionsformel II 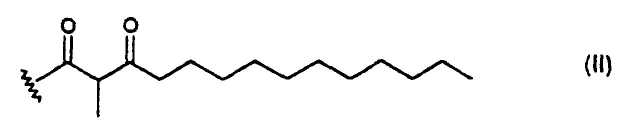 auf, wobei die bevorzugten Peptide Texenomycin A und B sich voneinander nur durch die Konfiguration (R- oder S-Konfiguration) des zwischen den beiden Carbonylgruppen angeordneten Kohlenstoffatoms bzw. durch die räumliche Anordnung der dieses Atom substituierenden Methylgruppe unterscheiden.

Die bevorzugten Texenomycine A und B werden vom Pilz Scleroderma texense, vozugsweise Scleroderma texense DSM 10601, gebildet und sind dadurch charakterisiert, daß sie die Summenformeln C₉₇H₁₇₀N₂₄O₂₃ (Texenomycin A) bzw. C₉₇H₁₇₀N₂₄O₂₃ (Texenomycin B) haben, ein UV-Maximum bei 280 nm haben (gemessen in Methanol) und und die Festpunkte von 215 -216 °C (Texenomycin A) bzw. 209 °C (Texenomycin B) haben.

Weiterhin gehören zum Gegenstand der vorliegenden Erfindung die Verfahren zur Herstellung der genannten Verbindungen. Ein Verfahren zur Herstellung der genannten Verbindungen ist dadurch gekennzeichnet, daß der Mikroorganismus Scleroderma texense, vorzugsweise Scleroderma texense DSM 10601, in einem wäßrigen Medium kultiviert wird und die Ziel verbindungen anschließend isoliert und gereinigt werden.

Der Mikroorganismus Scleroderma texense DSM 10601 ist am 20. März 1996 bei der Deutschen Sammlung von Mikroorganismen und Zellkulturen GmbH (DSMZ); Marscheroder Weg 1b, D-38124 Braunschweig nach den Vorschriften des Budapester Vertrags hinterlegt worden.

Anstelle des Stammes DSM 10601 können auch dessen Mutanten und Varianten eingesetzt werden, soweit sie Peptide der Formel I synthetisieren können. Solche Mutanten können in an sich bekannter Weise surch physikalische Mittel, beispielsweise Bestrahlung, wie mit Ultraviolett- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-Nnitrosoguanidin (MNNG) erzeugt werden.

Die vorliegende Erfindung betrifft demgemäß auch Scleroderma texense DSM 10601 sowie dessen Mutanten und Varianten.

Die im folgenden beschriebenen Fermentationsbedingungen gelten für Scleroderma texense und das hinterlegte Isolat DSM 10601. Die Fermentation kann im Labormaßstab (Milliliter- und Litermaßstab) oder im industriellen Maßstab (Kubikmetermaßstab) erfolgen.

In einer Nährlösung, die eine Kohlenstoffquelle und eine Stickstoffquelle sowie die üblichen anorganischen Salze enthält, produziert S. texense, insbesondere S. texense DSM 10601, die Peptide der Formel I.

Als bevorzugte Kohlenstoffquellen für aerobe Fermentation eigenen sich assimilierbare Kohlenhydrate und Zuckeralkohole, wie Glukose, Laktose oder D-Mannit sowie kohlenhydrathaltige Naturprodukte, wie z. B. Malzextrakt. Als stieksotffhaltige Nährstoffe kommen in Betracht: Aminosäuren, Peptide und Proteine sowie deren Abbauprodukte, wie Peptone onder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der Verbindung der Formel I mit S. texense, bevorzugt DSM 10601, verläuft besonders gut in einer Nährlösung, die 0,05 bis 5 %, bevorzugt 0,1 bis 2 %, Caseinpepton, 0,5 bis 7 %, bevorzugt 0,1 bis 2 % Fleischpepton, 0,1 bis 5 %, bevorzugt 0,5 bis 3 % Glucose, sowie 0,1 bis 5 %, bevorzugt 0,5 bis 3 % Maltose enthält, jeweils beogen auf das Gewicht der gesamten Nährlösung.

Die Kultivierung erfolgt aerob, also beispielsweise submers unter Schütteln oder Rühren oder in Fermentem, gegebenenfalls unter Einführen von Luft oder Wasserstoff. Sie wird in einem Temperaturbereich von 15 bis 30° C, insbesondere von 23 bis 28° C, durchgeführt werden. Der pH-Wert liegt von pH 1 bis 7, vorteilhaft von pH 2,5 bis 4,5. Man kultiviert den Pilz unter diesen Bedingungen über einen Zeitraum von 100 bis 300 Stunden, bevorzugt 120 bis 168 Stunden.

Vorteilhaft kultiviert man in mehreren Stufen, d. h.man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z. B. indem man Mycel in eine Nährlösung überimpft und etwa 100 bis 200 Stunden, bevorzugt 120 bis 168 Stunden, wachsen läßt. Das Mycel kann beispielsweise erhalten werden, indem man den Stamm 7 bis 40 Tage, bevorzugt 10 bis 20 Tage, auf einem festen oder flüssigen Nährboden, beispielsweise Hefe-Malz-Agar oder Kartoffel-Dextrose-Agar, wachsen läßt.

Der Fermentationsverlauf kann jeweils anhand des pH-Wertes der Kultur oder des Mycelvolumens sowie durch chromatographische Methoden, wie z. B. Dünnschichtchromatographie oder High Pressure Liquid Chromatography (HPLC), überwacht werden.

Die Texenomycine können sowohl im Mycel als auch im Kulturfiltrat vorkommen, gewöhnlich befindet sich die Hauptmenge in der Zallmasse. Es ist deshalb zweckmäßig, diese durch Filtration oder Zentrifugation vom Filtrat zu trennen. Das Filtrat wird lyophilisiert und das Lyophilisat mit einem Lösungmittel wie Methanol, Acetonitril oder 1-Butanol extrahiert. In gleicher Weise können Texenomycine aus dem Mycel extrahiert werden. Die Extraktionen können über einen weiten pH-Bereich durchgeführt werden, zweckmäßig ist ein Bereich von pH 3.0 - pH 7.5.

Eine andere Methode der Isolierung ist die Lösungs-Verteilung in an sich bekannter Weise.

Eine weitere Methode der Reinigung ist die Chromatographie an Adsorptionsharzen wie z.B. an Diaion® HP-20 (Mitsubishi Casei Corp., Tokyo) an Amberlite® XAD 7 (Rohm und Haas, USA) an Amberchrom® CG (Toso Haas, Philadelphia, USA) oder an ähnlichen Trägem. Geeignet sind darüberhinaus zahlreiche reversed-phase Träger, z.B. RP-18, wie sie in der Hochdruckflüssigkeits-Chromatographie (HPLC) allgemein verwendet werden. Eine weitere Reinigungsmöglichkeit für de Texenomycine besteht in der Verwendung sogenannter straight-Phase Träger wie z. B. Kieselgel oder Aluminiumoxid. Geeignet zur Elution sind viele Lösungsmittel wie z.B. Chloroform/Methanol/Eisessig Gemische.

Ein alternatives Verfahren zur Isolierung der Texenomycine ist die Verwendung sog. Molekularsiebe, wie z.B. Fractogel® TSK HW-40, Sephadex® LH-20. Es ist darüberhinaus auch möglich, die Texenomycine aus angereichertem Material durch Kristallisation zu gewinnen. Geeignet hierzu sind z.B. organische Lösungsmittel und ihre Gemische, wasserfrei oder mit Wasserzusatz. Ebenso können Zusätze von Säuren, wie zB. Trifluoressigsäure, Schwefelsäure, Salzsäure, Ameisensäure oder andere von Vorteil sein.

Es wurde gefunden, daß die Texenomycine das Wachstum verschiedener humanpathogener Pilze hemmen, z.B. Candida albicans mit einer MHK (Minimale Hemm Konzentration) von 0,24 µg/mL (Tex. A) bzw. 0,49 µg/mL (Tex. B).

Die erfindungsgemäßen Substanzen eignen sich zur Therapie und Prophylaxe von Pilzinfektionen:

Die vorliegende Erfindung betrifft daher auch ein Peptid der Formel I zur Verwendung als Arzneimittel und im besonderen ein Arzneimittel enthaltend mindestens ein Peptid gemäß Formel I und Arzneihilfsstoffe.

Die Erfindung betrifft insbesondere die Verwendung der Texenomycine oder seiner Derivate zur Herstellung eines Arzneimittels zur Behandlung von Pilzinfektionen.

### Beispiele

### 1. a) Herstellung von Mycel des Produzentenstammes S. texense DSM 10601

100 ml Nährlösung (20 g Malzextrakt, 2 g Hefeextrakt, 10 g Glucose, 0,5 g (NH₄)₂ HPO₄ in 1 l Leitungswasser, pH-Wert vor der Sterilisation 6,0) in einem 500 ml sterilen Erlenmeyerkolben werden mit dem Stamm DSM 10601 beimpft und 240 Stunden bei 25° C und 140 UpM auf einer rotierenden Schüttelmaschine inkubiert. Anschließend werden 20 ml Kulturflüssigkeit in einem sterilen 500 ml Erlenmeyerkolben mit dem Nährboden [Kartoffelinfus 4,0 g/l (Infus aus 200 g Kartoffeln in 1000 ml H₂O), 20,0 g D-Glucose, pH vor der Sterilisation 5,6], dem zusätzlich 15 g Agar/l zur Verfestigung zugegeben wurde, gleichmäßig verteilt und dekantiert. Die Kulturen werden 10 bis 21 Tage bei 25° C inkubiert.

Das nach dieser Zeit entstandene Mycel eines Kolben wird mit einer sterilen Impfnadel abgenommen, sofort weiterverwendet oder bei 4° C in Wasser aufbewahrt.

### 1. b) Herstellung einer Kultur bzw einer Vorkultur des Produzentenstammes im Erlenmeyerkolben

Ein steriler 500 ml Erlenmeyerkolben mit 100 ml der unter a) beschriebenen Nährlösung wird mit einer auf einem Schragrohrchen gewachsenen Kultur oder einem 1 qcm großen Mycelstück angeimpf und auf einer Schüttelmaschine bei 140 UpM und 25° C inkubiert. Die maximale Produktion einer Verbindung der Formal I ist nach ca. 168 Stunden erreicht. Zum Animpfen von 10- und 100-I Fermentem genügt eine 72 Stunden alte Submerskultur (Animpfmenge ca. 5 %) aus der gleichen Nährlösung.

### 2. Herstellung der Verbindung der allgemeinen Formel I

Ein 10 L Fermenter wird unter folgenden Bedingungen betrieben:

| | |
|---|---|
| 5 g/l | Caseinpepton |
| 10 g/l | Maltose |
| 5 g/l | Fleischpepton |
| 10 g/l | Glucose |
| pH 6,5 | (vor der Sterilisation) |

Der pH-Wert wird mit wäßriger 2N NaOH oder HCl eingestellt.

| | |
|---|---|
| Inkubationszeit | 168 Stunden |
| Inkubationstemperatur | 25° C |
| Rührergeschwindigkeit | 200 UpM |
| Belüftung | 5 l Luft/min |

Durch wiederholte Zugabe weniger Tropfen ethanolischer Polyollösung kann die Schaumbildung unterdrückt werden. Das Produktionsmaximum wird nach ca. 96 Stunden erreicht.

### 3. Isolierung der Texenomycine

8 L der nach Beispiel 2 gewonnenen Kulturlösung werden abzentrifugiert und die Zellmasse wird gefriergetrocknet. 35 g des Lyophilisats werden zweimal mit je 600 mL Methanol ausgerührt und jeweils über grobporiges Filterpapier abgenutscht. Das Filtrat wird vom Lösungsmittel befreit und nach Zusatz von Wasser lyophilisiert. 8 g des Lyophilisats werden mit Methanol digeriert und erneut filtriert.

### 4. Reinigung der Texenomycine

150 mL der methanolischen Lösung aus Beispiel 3 werden an Fractogel® TSK HW-40 (Säulenvolumen 2,5 L) mit Methanol (Fluß 40 mL/min) chromatographiert. Die Texenomycin-A-haltigen Fraktionen werden vereinigt und im Vakuum eingeengt.

Das angereicherte Material wird in Acetonitril/Methanol aufgeschlämmt, über Cellulosefilter filtriert, mit Wasser auf 30 % Lösungsmittelanteil verdünnt und an Merck RP-select B mit einem Acetonitril-Wasser Gradienten (0,05 % Trifluoressigsäure, 50 bis 100 Acetonitril) chromatographiert (Säulenabmessungen 250 x 20 mm, Fluß 23 mL/min). Die Texenomycin A enthaltenden Fraktionen werden vereinigt, im Vakuum konzentriert und lyophilisiert (45 mg Texenomycin A als weißes Pulver). Texenomycin B wird analog aus Texenomycin B-haltigen Fraktionen nach Fractogel® TSK HW-40 erhalten.

Die Struktur der erhaltenen Texenomycine wurde durch NMR-Spektroskopie bestimmt.

### 5. Antifungisches Wirkungsspektrum von Texenomycin A + B in Mikrodilutionstests

| | **MHK (µg/ml)** | |
|---|---|---|
| **Testkeim** | **Texenomycin A** | **Texenomycin B** |
| Candida albicans | 0,24 | 0,49 |
| Aspergillus fumigatus | 0,24 | 0,49 |
| Microsporum canis | 0,49 | 7,81 |
| Trichophyton mentagrophytes | 7,81 | 15,6 |
| Trichophyton rubrum | 31,25 | 31,25 |
| MHK = Minimale Hemmkonzentration | | |

Antibakterielles und antifungisches Wirkungsspektrum von Texenomycin A und B, eingesetzte Konzentration: 1 mg/ml

| **Testkeim** | **Agardiffusionstest [mm Hemmhof]** | |
|---|---|---|
| | **Texenomycin A** | **Texenomycin B** |
| Candida albicans | 20 | 20 |
| Saccharomyces cerevisiae | 19 | 19 |
| Aspergillus niger | 17 | 17 |
| Staph. P209 | 15 | 14 |
| Bacillus subtilis | 15 | 15 |
| Escherichia coli | - | - |
| Micrococcus luteus | 13 | 13 |
| Pseudomonas fluorescens | - | - |
| Streptomyces murinus | Spur | 10 |
| keine Aktivität: 7 mm | | |

## Patentansprüche

1. Peptid der Aminosäuresequenz I worin bedeuten
FS einen Oxo-Fettsäurerest,
Aib Aminoisobuttersäure und
Arg-ol Argininol.

2. Peptid nach Anspruch 1, **dadurch gekennzeichnet, daß** der Oxo-Fettsäurerest aus 3 bis 20 Kohlenstoffatomen besteht.

3. Peptid nach Anspruch 2, **dadurch gekennzeichnet, daß** der Oxo-Fettsäurerest aus 8 bis 15 Kohlenstoffatomen besteht.

4. Peptid nach Anspruch 3, **dadurch gekennzeichnet, daß** sich die Oxo-Gruppe des Oxo-Fettsäurerestes in 3-Position befindet.

5. Peptid nach Anspruch 4, **dadurch gekennzeichnet, daß** der Oxo-Fettsäurerest die Konstitutionsformel II 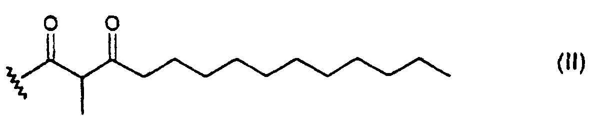 aufweist.

6. Peptid nach Anspruch 5, **dadurch gekennzeichnet, daß** der Fettsäurerest eine R-Konfiguration besitzt.

7. Peptid nach Anspruch 5, **dadurch gekennzeichnet, daß** der Fettsäurerest eine S-Konfiguration besitzt.

8. Verfahren zur Herstellung einer Verbindung gemäß einem der Ansprüche 1 bis 7, **dadurch gekennzeichnet, daß** Scleroderma texense in einem wäßrigen Medium kultiviert wird und die Zielverbindung anschließend isoliert und gereinigt wird.

9. Verfahren nach Anspruch 8, **dadurch gekennzeichnet, daß** Scleroderma texense DSM 10601 eingesetzt wird.

10. Verfahren nach Anspruch 9, **dadurch gekennzeichnet, daß** Mutanten oder Varianten von Scleroderma texense DSM 10601 eingesetzt werden.

11. Peptid gemäß einem der Ansprüche 1 bis 7 zur Verwendung als Arzneimittel.

12. Verwendung eines Peptids gemäß einem der Ansprüche 1 bis 7 zur Herstellung eines Arzneimittels zur Behandlung von Pilzinfektionen.

13. Arzneimittel enthaltend mindestens ein Peptid gemäß einem der Ansprüche 1 bis 7 und pharmazeutische Hilfsstoffe.

14. Scleroderma texense DSM 10601 sowie dessen Mutanten und Varianten.

## Claims

1. A peptide of amino acid sequence I in which
FS is an oxo fatty acid radical,
Aib is aminoisobutyric acid and
Arg-ol is argininol.

2. A peptide as claimed in claim 1, wherein the oxo fatty acid radical consists of 3 to 20 carbon atoms.

3. A peptide as claimed in claim 2, wherein the oxo fatty acid radical consists of 8 to 15 carbon atoms.

4. A peptide as claimed in claim 3, wherein the oxo group of the oxo fatty acid radical is found in the 3-position.

5. A peptide as claimed in claim 4, wherein the oxo fatty acid radical has the constitutional formula II

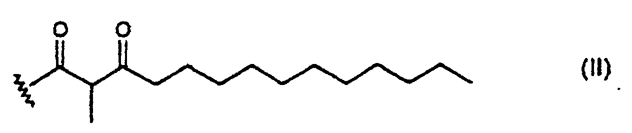

6. A peptide as claimed in claim 5, wherein the fatty acid radical has an R configuration.

7. A peptide as claimed in claim 5, wherein the fatty acid radical has an S configuration.

8. A process for the preparation of a compound as claimed in any of claims 1 to 7, which comprises culturing Scleroderma texense in an aqueous medium and then isolating and purifying the target compound.

9. The process as claimed in claim 8, wherein Scleroderma texense DSM 10601 is employed.

10. The process as claimed in claim 9, wherein mutants or variants of Scleroderma texense DSM 10601 are employed.

11. A peptide as claimed in any of claims 1 to 7 for use as a pharmaceutical.

12. The use of a peptide as claimed in any of claims 1 to 7 for the manufacture of a pharmaceutical for treating fungal infections.

13. A pharmaceutical comprising at least one peptide as claimed in any of claims 1 to 7 and pharmaceutical auxiliaries.

14. Scleroderma texense DSM 10601 and its mutants and variants.

## Revendications

1. Peptide ayant la séquence d'aminoacides I dans laquelle
FS représente un reste d'oxoacide gras,
Aib représente l'acide aminoisobutyrique et
Arg-ol représente l'argininol.

2. Peptide selon la revendication 1, **caractérisé en ce que** le reste d'oxoacide gras est constitué de 3 à 20 atomes de carbone.

3. Peptide selon la revendication 2, **caractérisé en ce que** le reste d'oxoacide gras est constitué de 8 à 15 atomes de carbone.

4. Peptide selon la revendication 3, **caractérisé en ce que** le groupe oxo du reste d'oxoacide gras se trouve en position 3.

5. Peptide selon la revendication 4, **caractérisé en ce que** le reste d'oxoacide gras présente la formule structurale II

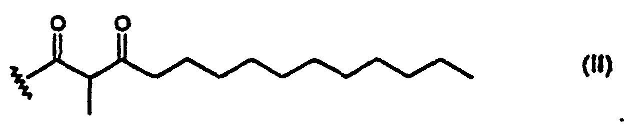

6. Peptide selon la revendication 5, **caractérisé en ce que** le reste d'acide gras a une configuration R.

7. Peptide selon la revendication 5, **caractérisé en ce que** le reste d'acide gras a une configuration S.

8. Procédé de préparation d'un composé selon l'une des revendications 1 à 7, **caractérisé en ce que** l'on cultive *Scleroderma texense* dans un milieu aqueux, puis on isole et on purifie le composé désiré.

9. Procédé selon la revendication 8, **caractérisé en ce que** l'on utilise *Scleroderma texense* DSM 10601.

10. Procédé selon la revendication 9, **caractérisé en ce que** l'on utilise des mutants ou des variantes de *Scleroderma texense* DSM 10601.

11. Peptide selon l'une des revendications 1 à 7, à utiliser comme médicament.

12. Utilisation d'un peptide selon l'une des revendications 1 à 7 pour la préparation d'un médicament destiné au traitement d'infections fongiques.

13. Médicament contenant au moins un peptide selon l'une des revendications 1 à 7 et des agents auxiliaires pharmaceutiques.

14. *Scleroderma texense* DSM 10601 et ses mutants et variantes.
